# EUROPÄISCHE PATENTSCHRIFT

(11) **EP 2 563 248 B1**
(45) Veröffentlichungstag und Bekanntmachung des Hinweises auf die Patenterteilung: **19.10.2016**
(21) Anmeldenummer: 11718004.2
(22) Anmeldetag: 28.04.2011
(51) Int. Cl.: A61B 17/70, A61B 17/86

(54) **PEDIKELSCHRAUBE UND VORRICHTUNG ZUR STABILISIERUNG DER WIRBELSÄULE**
PEDICLE SCREW AND DEVICE FOR STABILIZING THE SPINAL COLUMN
VIS PÉDICULAIRE ET DISPOSITIF POUR STABILISER LA COLONNE VERTÉBRALE

(30) Priorität: 30.04.2010 DE 102010028423
(43) Veröffentlichungstag der Anmeldung: 06.03.2013
(73) Patentinhaber: Kraus, Kilian, 97440 Werneck (DE)
(72) Erfinder: Kraus, Kilian, 97440 Werneck (DE)
(74) Vertreter: Meissner Bolte Partnerschaft mbB
(86) Internationale Anmeldenummer: PCT/EP2011/056696
(87) Internationale Veröffentlichungsnummer: WO 2011/135013

(56) Entgegenhaltungen:
- WO-A1-2009/029928
- WO-A2-2008/089096
- WO-A2-2009/106733
- US-A1- 2006 200 131
- US-A1- 2009 312 804

## Beschreibung

Die Erfindung betrifft eine Pedikelschraube und eine Vorrichtung zur Stabilisierung der Wirbelsäule. Eine solche Vorrichtung kommt etwa dann zum Einsatz, wenn ein etwa aufgrund eines Bandscheibendefekts nicht mehr funktionsfähiger Wirbel an benachbarten Wirbeln fixiert werden soll. Dabei werden meist zwei Stäbe kreisrunder Querschnittsform, im folgenden Verbindungsstäbe genannt, unter Einhaltung eines seitlichen Abstands mit Hilfe sog. Pedikelschrauben, siehe beispielsweise WO2009/029928 A1, WO2008/089096 A2, US2006/200131,

WO2009/106733 und US2009/312804 A1, an den betreffenden Wirbeln befestigt. Die Pedikelschrauben besitzen einen Schraubenschaft und einen Schraubenkopf. Der Schraubenkopf weist an seinem dem Schraubenschaft zugewandten unteren Ende eine Kopfbasis auf, an der zwei sich nach oben längs der Mittellängsachse weg erstreckende Wandabschnitte angeformt sind. Die Richtung, in der sich die Kopf-Mittellängsachse erstreckt wird im Folgenden als X-Richtung bezeichnet. Die wandabschnitte umgrenzen eine den Schraubenkopf in einer rechtwinklig zur X-Richtung, in Y-Richtung, durchsetzende Ausnehmung, welche zur Aufnahme eines Verbindungsstabs dient. Die Ausnehmung mündet auch in die Kopfoberseite, wobei von dort zum Zwecke der Fixierung eines Verbindungsstabs eine ein Außengewinde aufweisende Fixierschraube in ein Innengewinde der Wandabschnitte eingeschraubt ist. Hinsichtlich der Anbindung des Kopfes an den Schraubenschaft unterscheidet man monoaxiale und polyaxiale Pedikelschrauben. Bei ersteren ist der Schraubenkopf starr mit dem Schraubenschaft verbunden, wobei der Schraubenschaft in Richtung der Mittellängsachse des Schraubenkopfes bzw. in X-Richtung verläuft. Sind solche Schrauben in einen fehlständigen Wirbel eingeschraubt, kann dieser mit Hilfe eines am Schraubenkopf angesetzten Werkzeugs leicht reponiert, also in seine normale Position gebracht werden.

Nachteil dieser Schrauben ist, dass beilspielsweise nach einer Reponierung eines Wirbels die Pedikelschraube eine schrägstellung gegenüber einem Verbindungsstab aufweist, so dass dieser entsprechend schräg und nicht gerade bzw. in Y-Richtung in der Ausnehmung des Schraubenkopfes verläuft und daher nicht zuverlässig darin fixierbar ist. Abhilfe schaffen hier die polyaxialen Pedikelschrauben, bei denen Schraubenkopf und Schraubenschaft über ein Kugelgelenk miteinander verbunden sind. Nachteilig ist aber, dass aufgrund der - polyaxialen - Beweglichkeit des Kopfes eine Reposition eines Wirbels auf die oben beschriebene Art und Weise nicht möglich ist. Hierzu ist ein Dornwerkzeug erforderlich, das in eine oberseits im Schraubenschaft vorhandene Axialöffnung eingesteckt wird. Einen Kompromiss zwischen beiden Schraubentypen bildet eine aus DE 10 2005 005 647 A1 bekannte Pedikelschraube, bei welcher der Kopf gegenüber dem Schraubenschaft nur um eine einzige, sich rechtwinklig zur X-Richtung verlaufenden Richtung, der Y-Richtung, schwenkbar ist. Der Schraubenkopf kann auf diese Weise in Bezug auf Y-Richtung bzw. die Längsrichtung eines Verbindungsstabs geschwenkt werden, um etwa den Abstand zwischen zwei Verbindungsstäben zu variieren. Gleichwohl kann aber der Schraubenkopf in Y-Richtung, also etwa in Richtung der Längserstreckung eines Verbindungsstabs zum Zwecke der Reposition eines Wirbels mit Hilfe eines Werkzeugs beaufschlagt werden, da er bezüglich dieser Richtung starr mit dem Schraubenschaft verbunden ist. Bei der bekannten Schraube wird die Verschwenkbarkeit des Verbindungstabes in der Ausnehmung des Schraubenkopfes durch eine dort vorhandene Klemmeinrichtung gewährleistet, wobei diese durch die o.g. Fixierschraube unter Festklemmen des Verbindungsstabs in Richtung auf die Kopfbasis mit einer Kraft beaufschlagt wird. Diese Kraft muss in den Schraubenschaft eingeleitet werden, damit die gelenkige Verbindung zwischen Schraubenkopf und Schraubenschaft blockiert und der Schraubenkopf in der jeweiligen Schwenklage fixiert ist. Damit der Verbindungsstab im Montagezustand, also bei festgezogener Fixierschraube, über eine gegebenenfalls sehr lange Zeitdauer zuverlässig gehalten ist, ist eine möglichst geringe Flächenpressung der miteinander zusammenwirkenden Teile anzustreben, d.h. der Kraftfluss in den Schraubenschaft soll über zwei möglichst großflächig aneinander liegenden Flächen erfolgen. Bei der bekannten Pedikelschraube stützt sich die Klemmeinrichtung mit einer Kugelfläche an einer stirnseitigen komplementären Kugelfläche des Schraubenschafts ab. Aufgrund der schwenkbaren Lagerung des Schraubenkopfes am Schraubenschaft existiert aber nur eine Schwenkstellung, bei der die miteinander zusammenwirkenden Kugelflächen konzentrisch zueinander sind. In allen anderen Schwenklagen des Kopfes ist dies nicht der Fall, so dass es zu einem punkt- oder allenfalls Kontakt zwischen den Kugelflächen kommt. Eine zuverlässige Fixierung des Verbindungsstabs ist dabei nicht gegeben.

Aufgabe der Erfindung ist es, eine Pedikelschraube und eine Vorrichtung zur Stabilisierung der Wirbelsäule vorzuschlagen, die in dieser Hinsicht verbessert ist.

Diese Aufgabe wird bei einer Pedikelschraube der eingangs genannten bzw. der im Oberbegriff des Anspruchs 1 wiedergegebenen Art durch die kennzeichnenden Merkmale des Anspruches 1 und hinsichtlich einer Vorrichtung durch Anspruch 17 gelöst. Gemäß Anspruch 1 ist in der Kopfbasis ein zwischenelement vorhanden, das auf seiner dem Klemmelement zugewandten Seite eine mit der unteren Kugelfläche der Klemmeinrichtung zusammenwirkende Gegen-Kugelfläche und auf seiner dem Schraubenschaft zugewandten Seite eine Zylinderfläche aufweist, die mit einer am Gelenkkopf vorhandenen GegenZylinderfläche zusammenwirkt, wobei die Zylinderflächen eine gemeinsame, mit der Schwenkachse des Schraubenkopfes zusammenfallende Krümmungsachse aufweisen und der Gelenkkopf und die Kopfbasis durch einen Bajonettverschlusses miteinander verbunden sind. Eine derartige Ausgestaltung gewährleistet, dass bei jeder Schwenkstellung des Schraubenkopfes die Krafteinleitung in den Schraubenschaft über eine großflächige Anlage der beteiligten Teile erfolgt. Bei einer Verschwenkung des Schraubenkopfes bleiben der gemeinsame Kugelmittelpunkt der Kugelflächen sowie die gemeinsame Krümmungsachse unverändert, so dass ein punkt- oder linienförmiger gegenseitiger Kontakt vermieden und eine zuverlässige Fixierung des Verbindungsstabs in jeder Schwenklage des Schraubenkopfes gewährleistet ist.

Vorteile der in den Unteransprüchen angegebenen Weiterbildungen gehen aus der folgenden Beschreibung hervor, welche auf die beigefügten Zeichnungen Bezug nimmt. Es zeigen:
- Fig. 1: eine perspektivische Explosionsdarstellung einer Pedikelschraube,
- Fig. 2: einen Querschnitt längs der in Fig. 1 gekennzeichneten XZ-Ebene,
- Fig. 3: den Schnitt gemäß Fig. 2 in perspektivischer Darstellung,
- Fig. 4: ein Zwischenelement in perspektivischer Darstellung,
- Fig. 5: eine perspektivische Darstellung des Zwischenelementes in einer gegenüber Fig. 4 veränderten Position,
- Fig. 6: eine Seitenansicht in Richtung des Pfeils VI in Fig. 4,
- Fig. 7: einen Längsschnitt einer Pedikelschraube, bei welcher der Schraubenkopf gegenüber dem Schraubenschaft verschwenkt ist,
- Fig. 8: einen Teillängsschnitt durch einen Schraubenkopf mit schräg darin gehaltenem Verbindungsstab,
- Fig. 9: eine perspektivische Darstellung einer Fixierschraube mit daran fixiertem oberen Klemmelement,
- Fig. 10: einen Längsschnitt-entsprechend der Linie X-X in Fig. 9, wobei das obere Klemmelement gegenüber der Fixierschraube verschwenkt ist,
- Fig. 11: eine perspektivische Ansicht des oberen Klemmelementes,
- Fig. 12: eine perspektivische Darstellung des oberen Klemmelementes in einer anderen Lage,
- Fig. 13: eine Draufsicht auf die Unterseite des Schraubenkopfes,
- Fig. 14: eine in Seitenansicht dargestellte Pedikelschraube, bei welcher die Mittellängsachse des Schraubenkopfes und die Mittellängsachse des Schraubenschaftes auf einer Linie liegen,
- Fig. 15: eine aus zwei Verbindungsstäben und sechs Pedikelschrauben gebildete Vorrichtung zur Stabilisierung einer Wirbelsäule,

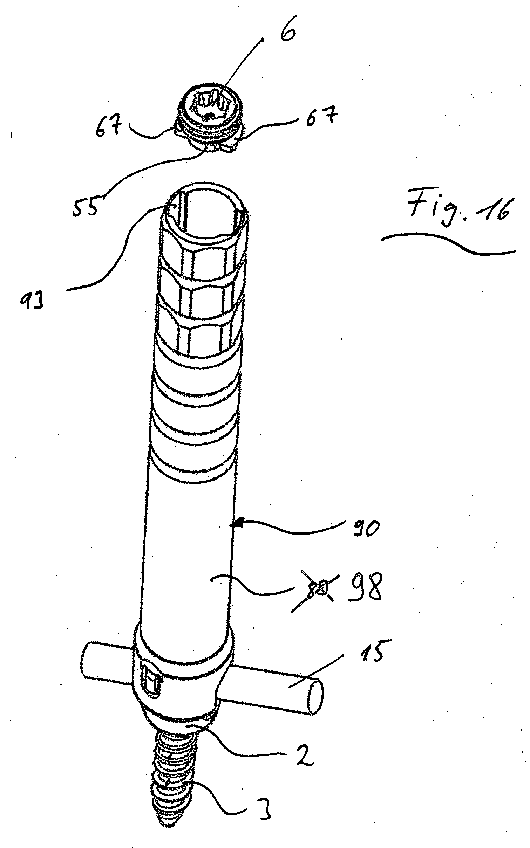

- Fig. 16: eine Schraube mit aufgesetztem Montagewerkzeug in perspektivischer Ansicht.

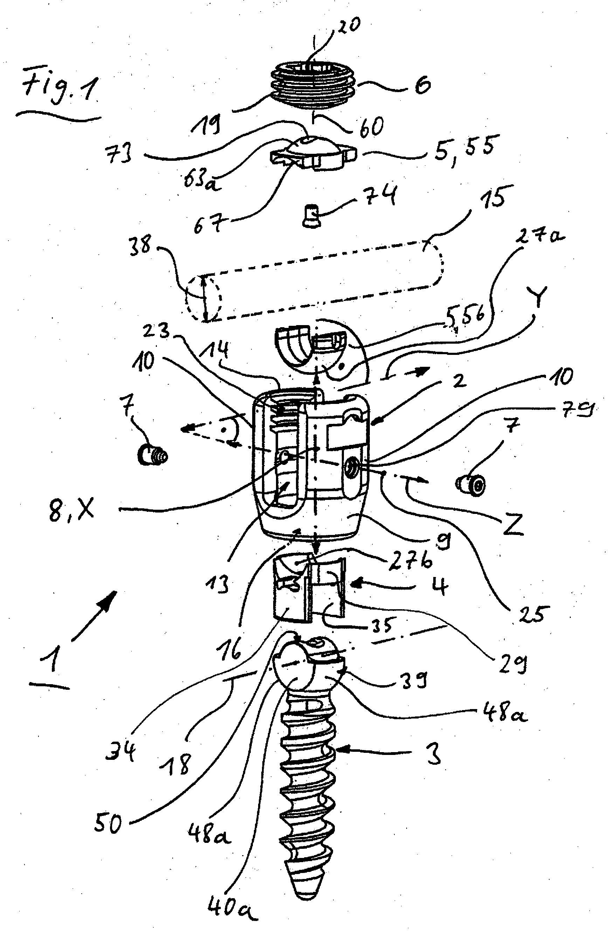

Wie am besten Fig. 1 zu entnehmen ist, setzt sich eine erfindungsgemäße Pedikelschraube im Wesentlichen aus einem Schraubenkopf 2, einem Schraubenschaft 3, einem Zwischenelement 4, einer Klemmeinrichtung 5, einer Fixierschraube 6 und zwei Lagerzapfen 7 zur schwenkbaren Lagerung der Klemmeinrichtung 5 im Schraubenkopf 2 zusammen. Die Richtung, in der sich die Mittellängsachse 8 des Schraubenkopfes 2 erstreckt, wird im folgenden als X-Richtung bezeichnet. Der Schraubenkopf 2 weist an seinem dem Schraubenschaft 3 zugewandten unteren Ende eine Kopfbasis 9 auf, an der zwei sich in X-Richtung nach oben bzw. vom Schraubenschaft 3 weg erstreckende Wandabschnitte 10 angeformt sind. Die Wandabschnitte 10 bilden eine seitliche Begrenzung für eine Ausnehmung 13, welche den Schraubenkopf 2 in Y-Richtung durchsetzt und welche sich in die Kopfoberseite 14 öffnet. Der Schraubenschaft 3 ragt über eine Bodenöffnung 16, die mit der Ausnehmung 13 in Verbindung steht, mit seinem kopfnahen bzw. oberen, als Schwenkkopf 39 ausgebildeten Ende in die Kopfbasis 9 hinein und ist darin so gelagert, dass der Schraubenkopf 2 gegenüber dem Schraubenschaft 3 um eine einzige, parallel zur y-Richtung verlaufende Kopfschwenkachse 18 schwenkbar ist. Die Öffnungsebene der in der Kopfbasis 9 vorhandenen Bodenöffnung 16 erstreckt sich quer zur X-Richtung.

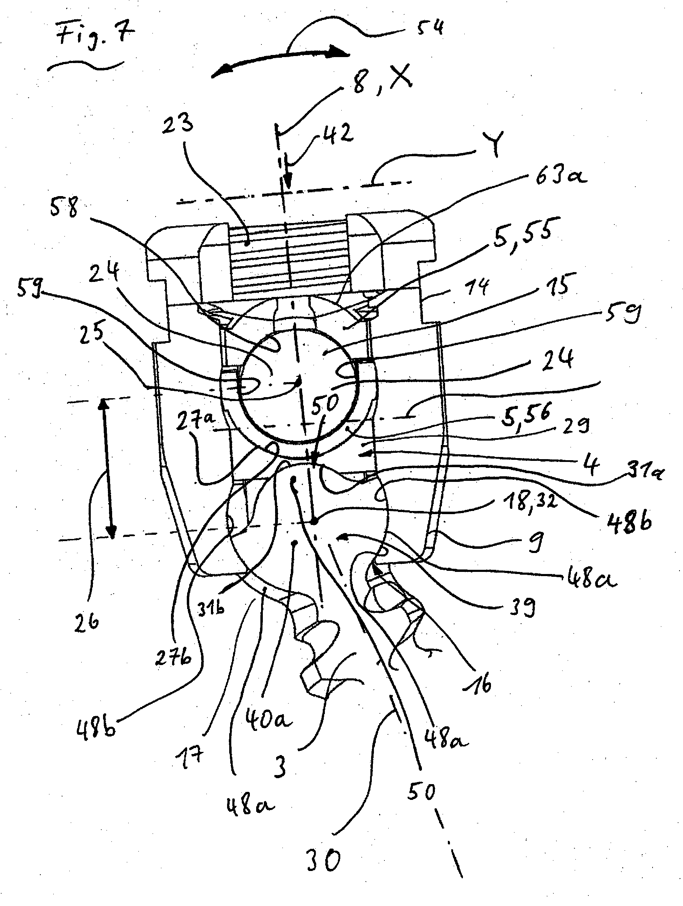

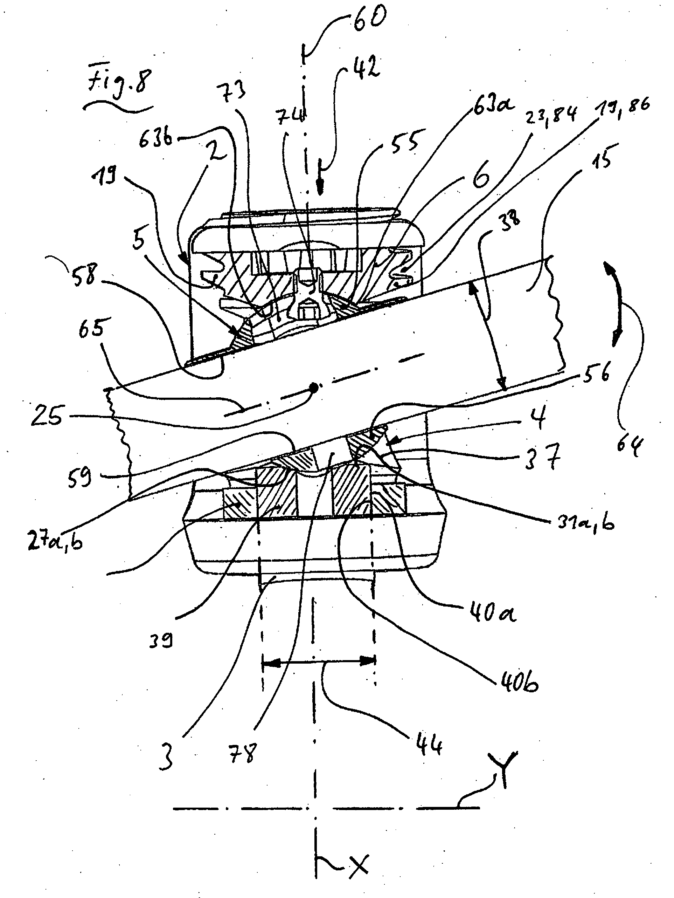

Die Fixierschraube 6 ist von der Kopfoberseite 14 her in die Ausnehmung 13 eingesetzt. Sie weist etwa die Form einer Madenschraube auf und umfasst ein Außengewinde 19. In ihrer dem Schraubenkopf 2 abgewandten Stirnseite ist eine Ausnehmung 20 vorhanden, in die ein Drehbetätigungswerkzeug (nicht gezeigt) einsteckbar ist. Das Außengewinde 19 greift in ein an den Freienden der Wandabschnitte 10 vorhandenes Innengewinde 23 ein. Die Klemmeinrichtung 5 ist im Montagezustand (Fig. 7, 8) zwischen der Fixierschraube 6 und der Kopfbasis 9 angeordnet und weist einen Hohlraum 24 auf, der vom kreiszylinderförmigen Verbindungsstab 15 durchsetzt ist. Weiterhin ist die Klemmeinrichtung 5 so im Schraubenkopf 2 gehalten, dass sie um eine Schwenkachse 25 schwenkbar ist. Die Schwenkachse 25 erstreckt sich in Z-Richtung, wobei diese rechtwinklig zur X- und Y-Richtung bzw. rechtwinklig zu der von diesen Richtungen aufgespannten Ebene (die Zeichenebene in Fig. 7) verläuft. Außerdem ist die Schwenkachse 25 näher an der Kopfoberseite 14 angeordnet als die Kopfschwenkachse 18, weist also einen Abstand 26 (Fig. 7) in X-Richtung zur Kopfschwenkachse 18 auf. Durch den Abstand 26 zwischen den genannten Achsen ist gewährleistet, dass ein von der Klemmeinrichtung 5 gehaltener Verbindungsstab 15 hinsichtlich der die Kopfschwenkachse 18 enthaltenden X-Z-Ebene, die sich in Fig. 7 senkrecht zur Papierebene erstreckt, seitlich verschwenkbar ist. Auf diese Weise lässt sich beispielsweise der Abstand zwischen zwei nebeneinander an der Wirbelsäule fixierten Verbindungsstäben 15 ändern. Im Montagezustand ist die Klemmeinrichtung 5, von der festgezogenen Fixierschraube 6 mit einer zur Kopfbasis 9 gerichteten Kraft beaufschlagt. Dabei wird eine an der Klemmeinrichtung 5 vorhandene untere Kugelfläche 27a gegen eine am Zwischenelement 4 vorhandene komplementäre Gegen-Kugelfläche 27b gedrückt. Die Kugelmittelpunkte der genannten Kugelflächen liegen, zumindest im Montagezustand, auf der Schwenkachse 25 der Klemmeinrichtung 5. Dadurch ist gewährleistet, dass unabhängig von der Schwenkstellung des Schraubenkopfes 2 ein vollflächiger gegenseitiger Kontakt der genannten Flächen vorhanden ist. Die Gegen-Kugelfläche 27b ist an der nach oben bzw. zur Fixierschraube 6 weisenden Seite des Zwischenelements 4 vorhanden.

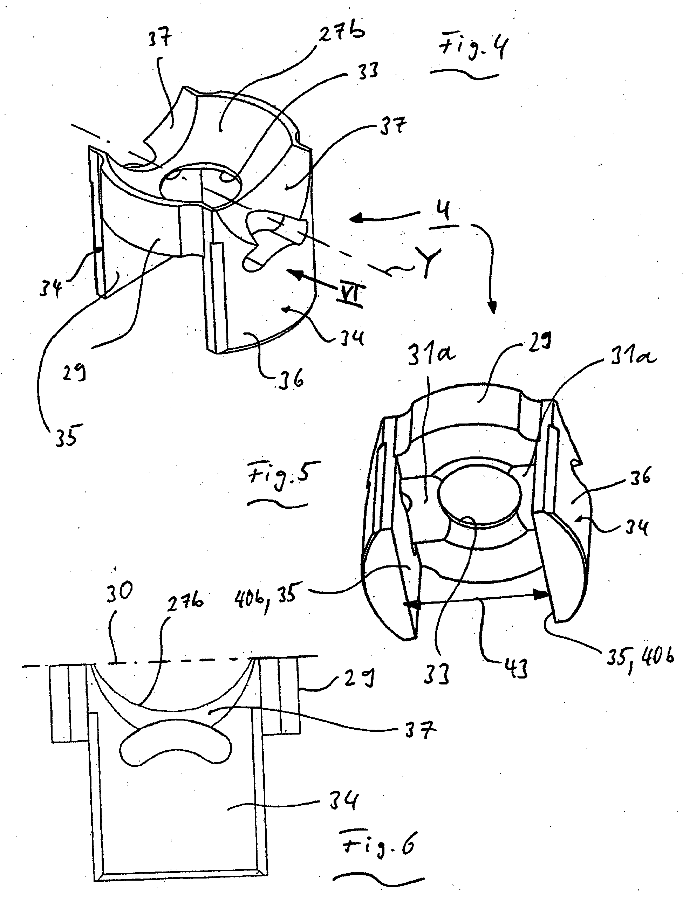

Das Zwischenelement 4 weist eine etwa scheibenförmige Basis 29 auf, deren Planebene 30 (Fig. 6) - im Montagezustand - sich parallel zu der von der Y- und Z-Richtung aufgespannten Ebene erstreckt. Die Gegen-Kugelfläche 27b ist an der Oberseite der Basis 29 vorhanden. Die Basis 29 ist von einer zentralen Öffnung 33 durchsetzt, welche der Montageerleichterung dient.

Seitlich in Y-Richtung sind an die Basis 29 zwei Wandabschnitte 34 angeformt, welche sich in X-Richtung nach unten bzw. zum Schraubenschaft 3 hin von der Basis 29 weg erstrecken. Die Innenseiten 35 der Wandabschnitte 34 sind planeben ausgebildet, wobei sie sich parallel zur X-Richtung und quer zur Y-Richtung erstrecken, d.h. sie verlaufen in der X-Z-Ebene. Die Außenseiten 36 der Wandabschnitte 34 sind mit kreisbogenförmiger Kontur radial nach außen gewölbt. Die Wandabschnitte 34 sind in Y-Richtung von einer Öffnung 41 durchbrochen, welche der Herstellungsvereinfachung dient. Die oberen Stirnseiten 37 der Wandabschnitte 34 verlaufen schräg nach unten und sind Teile der Mantelfläche eines gedachten Zylinders, dessen Durchmesser dem Durchmesser 38 des Verbindungsstabes 15 entspricht. Durch die abgeschrägten Stirnseiten 37 wird der Schwenkbereich eines Verbindungsstabes 15 in der Ausnehmung 13 des Schraubenkopfes 2 vergrößert, wobei in den jeweiligen Schwenk-Endlagen der Verbindungsstab 15 auf den Stirnseiten 37 aufliegt. An der dem Schraubenschaft 3 zugewandten Seite bzw. an der Unterseite der Basis 29 ist eine Zylinderfläche 31a angeordnet, deren Krümmungsachse 32 sich in Z-Richtung erstreckt (Fig. 7).

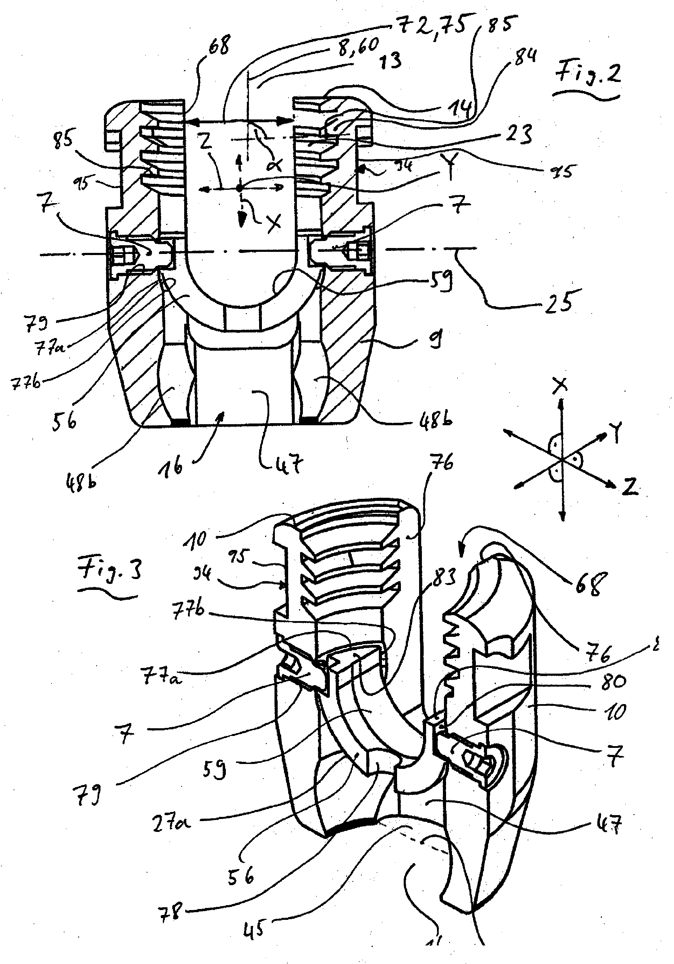

Der Gelenkkopf 39 weist zwei diametral gegenüberliegende Kugelflächen 48a auf, die mit komplementären Gegenkugelflächen 48b an der die Bodenöffnung 16 umgrenzenden Wand der Kopfbasis 9 im Sinne eines Kugelgelenks zusammenwirken (Fig. 7). Außerdem sind am Gelenkkopf 39 zwei parallel zueinander und parallel zur X-Z-Ebene verlaufende Planflächen 40a vorhanden, die zur Fixierung des Gelenkkopfs hinsichtlich einer Drehung um die Mittellängsachse 30 des Schraubenschafts 3 mit Gegen-Planflächen 40b zusammenwirken. Diese könnten an der Wand der Bodenöffnung 16 vorhanden sein. Bevorzugt ist jedoch, dass die Gegen-Planflächen 40b von den Innenseiten 35 der Wandabschnitte 34 des Zwischenelements 4 gebildet sind. Die lichte Weite 43 (Fig. 5) zwischen den Innenseiten 35 der Wandabschnitte 34 ist geringfügig größer als die über die Planflächen 40a gemessene Breite 44 (Fig. 8) des Gelenkkopfes 39, so dass dieser im Wesentlichen spielfrei von den Wandabschnitten 34 umklammert ist. Die Planflächen 40a wirken dabei mit den Innenseiten 35 bzw. den Gegen-Planflächen 40b im Sinne einer Gleitpaarung zusammen. Aufgrund dieser Gleitpaarung ist die Schwenkbewegung des Schraubenkopfes 2 gegenüber dem Schraubenschaft 3 auf eine einzige Achse, nämlich die Schwenkachse 18, beschränkt. Die Wandabschnitte 34 ragen in einen in Z-Richtung seitlich erweiterten Bereich 45 der Bodenöffnung 16 des Schraubenkopfes 2, der in Fig. 3 durch die gestrichelte Linie 46 angedeutet ist, formschlüssig hinein. Durch den Formschluss ist verhindert, dass sich der Schraubenschaft 3 um seine Mittellängsachse 30 drehen kann. Der radial erweiterte Bereich 45 ist in Y-Richtung durch einen kreisbogenförmig nach außen gekrümmten Innenwandbereich 47 der Kopfbasis 9 begrenzt (siehe Fig. 2 und 3). Die Gegen-Kugelflächen 48b an der Wand der Bodenöffnung 16 erstrecken sich so weit in X-Richtung nach oben und nach unten, dass der Gelenkkopf 39 in der genannten Richtung im Schraubenkopf 2 fixiert ist.

Auf der der Kopfoberseite 14 zugwandten Seite des Gelenkkopfes 39 ist ein Vorsprung 50 vorhanden, dessen der Kopfoberseite 14 zugewandter Oberflächenbereich zumindest in einem mittleren Bereich als eine Zylinderfläche 31b ausgebildet ist, wobei diese mit der an der Unterseite der Basis 29 des Zwischenelements 4 vorhandenen Zylinderfläche 31a im Sinne einer Gleitpaarung zusammenwirkt. Die Krümmungsachse 32 der Zylinderflächen 31a, 31b fällt mit der Kopfschwenkachse 18 zusammen.

Aufgrund der beschriebenen Ausgestaltung bleiben die genannten Zylinderflächen bei jeder Schwenkstellung des Schraubenkopfes 2 großflächig in Kontakt, so dass die von der Fixierschraube 6 in X-Richtung nach unten bzw. in Richtung des Pfeils 42 in Fig. 7 auf die Klemmeinrichtung 5 einwirkende Kraft großflächig in den Gelenkkopf 39 eingeleitet wird. Dabei werden dessen Kugelflächen 48a gegen die Gegen-Kugelflächen 48b der Kopfbasis 9 gedrückt und der Schraubenkopf 2 in der jeweiligen Schwenkstellung am Schraubenschaft 3 fixiert.

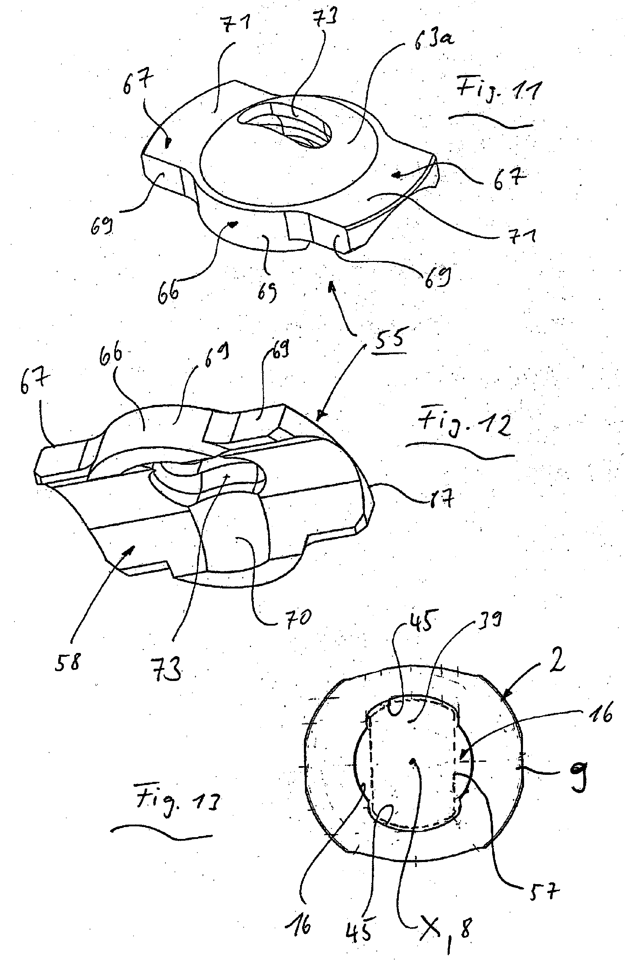

Der Gelenkkopf 39 des Schraubenschaftes 3 und die Kopfbasis 9 sind durch einen Bajonettverschlusse miteinander verbunden. Dies wird dadurch erreicht, dass die Bodenöffnung 16 eine derartige Querschnittform aufweist, dass der Gelenkkopf 39 in einer ersten Drehstellung in die Kopfbasis einführbar ist. Diese Situation ist in Fig. 13 dargestellt. Der Umriss des Gelenkkopfes 39 ist durch die gestrichelte Linie 57 angedeutet. Es ist erkennbar, dass der Gelenkkopf 39 in X-Richtung bzw. in Richtung der Mittellängsachse 8 des Schraubenkopfes in die Bodenöffnung 16 einführbar ist. Wenn sich der Gelenkkopf 39 innerhalb der Kopfbasis 9 befindet, bewirkt eine Verdrehung des Schraubenschafts 3 gegenüber der in Fig. 13 gezeigten Position um 90° eine Verriegelung des Gelenkkopfes 39 in der Kopfbasis 9. In dieser Situation liegen die Kugelflächen 48a, des Zylinderkopfes 39 an den Gegenkugelflächen 48b an der Wand der Bodenöffnung an (Fig. 7).

Die Klemmeinrichtung 5 setzt sich aus einem oberen Klemmelement 55 und einem unteren Klemmelement 56 zusammen, wobei diese Elemente Klemmflächen 58,59 aufweisen, welche im Montagezustand konzentrisch zur Schwenkachse 25 der Klemmeinrichtung 5 verlaufen und deren Hohlraum 24 zumindest teilweise umgrenzen. Der Durchmesser 72 des Hohlraums 24 entspricht der lichten Weite 75 zwischen den Seitenkanten 76 der Wandabschnitte 10.

Das obere Klemmelement 55 ist an der Fixierschraube 6 um deren Mittellängsachse 60 drehbar und ansonsten axial fest gehalten. Das obere Klemmelement 55 weist weiterhin eine vom Schraubenschaft 3 weg weisende obere Kugelfläche 63a auf, welche mit einer komplementär gestalteten Gegenkugelfläche 63b an der Unterseite der Fixierschraube 6 zusammenwirkt. Die genannten Flächen werden im Montagezustand gegeneinander gedrückt und weisen dann einen gemeinsamen, auf der Schwenkachse 25 der Klemmeinrichtung 5 liegenden Kugelmittelpunkt auf. Bei noch nicht ganz fest gezogener Fixierschraube 6 kann ein von den Klemmelementen 55,56 gehaltener Verbindungsstab 15 in der X-Y-Ebene, etwa entsprechend Doppelpfeil 64 in Fig. 8 verschwenkt werden. Die Schwenkachse 25 der Klemmeinrichtung schneidet deren Hohlraum 24 zumindest im Montagezustand mittig, verläuft somit durch die Mittellängsachse 65 des Verbindungsstabes 15.

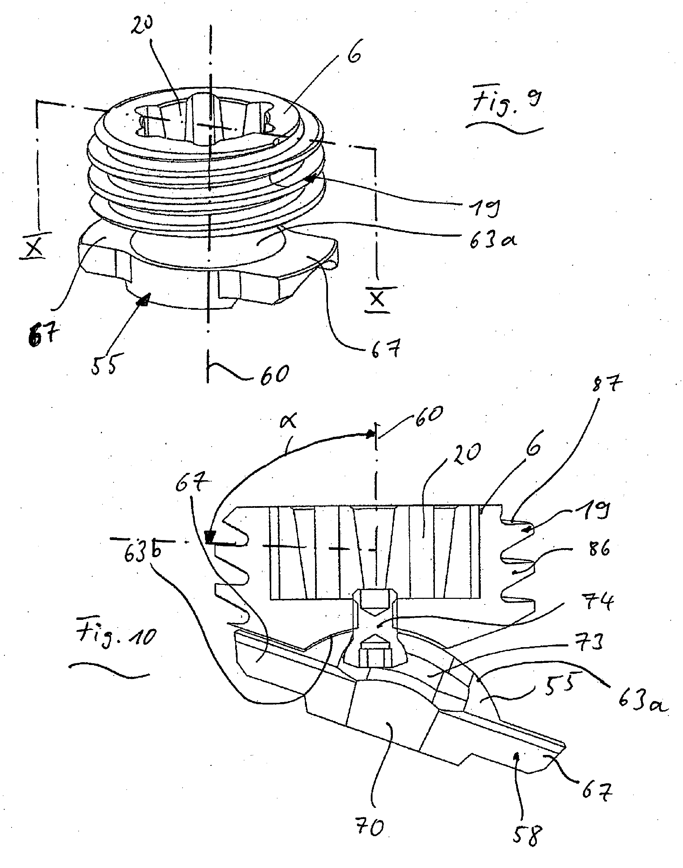

Das obere Klemmelement 55 umfasst einen zentralen, in der Draufsicht etwa kreisrunden Bereich 66, der in die Ausnehmung 13, d.h. zwischen die Wandabschnitte 10 des Schraubenkopfes, deren Innenseiten eine dem Bereich 66 entsprechende Rundung aufweisen, einführbar ist. Das obere Klemmelement 55 ist drehfixiert in der Ausnehmung 13 des Schraubenkopfes 2 gehalten. Dadurch ist gewährleistet, dass sich das obere Klemmelement 55 bei der Fixierung eines Verbindungsstabes 15, also wenn die Fixierschraube 6 in das Innengewinde 23 des Schraubenkopfes 2 eingedreht wird, nicht verdrehen kann und versehentlich in dieser verdrehten Position gegen den Verbindungsstab 15 gedrückt wird. Die Drehsicherung des oberen Klemmelementes 55 wird durch zwei diametral gegenüberliegende, etwa lappenförmige, an Bereich 66 angeformte Fortsätze 67 erreicht, welche mit einem in Umfangsrichtung des Schraubenkopfes 2 wirksamen Formschluss in den zwischen den Wandabschnitten 10 vorhandenen Zwischenraum 68 (Fig. 2 und 3) hinein ragen. Die Oberseiten 71 der Fortsätze 67 verlaufenen in einer gemeinsamen Planebene, aus der sich die Kugelfläche 63a hervor wölbt. Sowohl die Fortsätze 67 als auch der kreisscheibenförmige Bereich 66 weisen Seitenwände 69 auf, die sich rechtwinklig zu der von dem oberen Klemmelement 55 bzw. von den Oberseiten 71 der Fortsätze aufgespannten Planebene, im Montagezustand somit in X-Richtung erstrecken. Die Fortsätze 67 sind so bemessen, dass sie aus den zwischen den Wandabschnitten 10 vorhandenen Zwischenräumen 68 nicht herausragen. Die sich an der Unterseite des Klemmelementes 55 befindliche Klemmfläche 58 ist komplementär zur Oberfläche eines Verbindungsstabes ausgebildet. Sie ist mittig von einer beispielsweise kugelförmigen Ausnehmung 70 unterbrochen. Zentral in der Ausnehmung 70 bzw. in der Klemmfläche 58 ist ein Langloch 73 vorhanden, das in der oberen Kugelfläche 63a ausmündet. Das Langloch 73 erstreckt sich in Richtung der Fortsätze 67 bzw. im Montagezustand in Y-Richtung und gewährleistet eine Verschwenkbarkeit des Klemmelementes 55 an der Fixierschraube 10 (siehe Fig. 10). Die axialfeste Verbindung zwischen Fixierschraube 6 und Klemmelement 55 erfolgt über eine das Langloch 73 durchsetzende Schraube 74. Die erwähnte Ausnehmung 70 im Klemmelement 55 bewirkt, dass dieses im Montagezustand in X-Richtung eine Federwirkung hat, was sich günstig auf die Zuverlässigkeit der Fixierung eines Verbindungsstabes 15 auswirkt.

Das untere Klemmelement 56 ist halbringförmig ausgestaltet, wobei an seiner im Montagezustand dem Schraubenschaft 3 zugewanden Außenseite die untere Kugelfläche 27a der Klemmeinrichtung 5 angeordnet ist. Die Freienden des Klemmelementes 56 sind radial verbreitert und tragen an ihrer Außenseite eine Kugelfläche 77a, die mit einer Gegen-Kugelfläche 77b an der Innenseite der Wandabschnitte 10 zusammenwirkt. Die genannten Kugelflächen verlaufen allesamt konzentrisch, d.h. sie haben einen gemeinsamen Kugelmittelpunkt, wobei dieser auf der Schwenkachse 25 der Klemmeinrichtung 5 liegt. Das Klemmelement 56 ist mittig von einer in Richtung der Mittellängsachse 8 des Schraubenkopfes 2 verlaufenden Bohrung 78 durchsetzt, welche den Zusammenbau der Pedikelschraube 1 aus ihren Einzelteilen erleichtert. Das Klemmelement 56 ist drehfixiert im Schraubenkopf 2 gelagert. Diese Drehfixierung wird durch die eingangs schon erwähnten Lagerzapfen 7 gewährleistet, welche in seitliche, sich konzentrisch zur Schwenkachse 25 erstreckende Bohrungen 79 eingesetzt sind. Die Lagerzapfen ragen aus den Innenseiten der Wandabschnitte 10 des Schraubenkopfes 2 in der genannten Richtung vor und greifen jeweils in ein in den Freienden des Klemmelementes 56 vorhandenes Langloch 80 ein. Das Langloch 80 ist in mittiger Position an den Freienden des Klemmelementes 56 angeordnet, erstreckt sich etwa in dessen Umfangsrichtung und mündet in dessen Stirnseite 83 aus. Wenn in einem Vormontagezustand, in dem der Verbindungsstab 15 der Klemmeinrichtung 5 nicht mit der endgültigen Festigkeit gehalten ist, weil die Fixierschraube 6 nur leicht angezogen ist, kann das Klemmelement 56 um seine Schwenkachse 25 verschwenkt werden. Da es noch nicht an die Gegen-Kugelfläche 27b des Zwischenelementes 4 gepresst ist, ist dies mit relativ geringem Kraftaufwand möglich. Dabei ist zwischen der Kugelfläche 27a und der Gegen-Kugelfläche 27b ein geringfügiger Spalt vorhanden. Im Montagezustand muss dagegen das untere Klemmelement 56 fest an das Zwischenelement 4 gepresst werden, muss also im Vergleich zum Vor-Montagezustand ein geringfügig nach unten bewegt werden, was durch die oben beschriebene Lagerung über Lagerzapfen 7 und Langloch 80 gewährleistet ist.

Das Innengewinde 23 des Schraubenkopfes 2 und das Außengewinde 19 der Fixierschraube 6 sind so ausgestaltet, dass beim Festziehen der Fixierschraube 6 die Wandabschnitte 10 radial nach innen gezogen werden. Dies wird dadurch erreicht, dass das Innengewinde 23 des Schraubenkopfes 2 einen Gewindezahn 84 mit einer nach unten weisenden Flanke 85 und das Außengewinde 19 der Fixierschraube 6 einen Gewindezahn 86 mit einer nach oben weisenden Flanke 87 aufweist. Die erstgenannte Flanke 85 schließt mit der Mittellängsachse 8 des Schraubenkopfes 2 und die letztgenannte Flanke 87 mit der Mittellängsachse 60 der Fixierschraube 6 einen sich nach oben öffnenden spitzen Winkel α, beispielsweise von 87° ein.

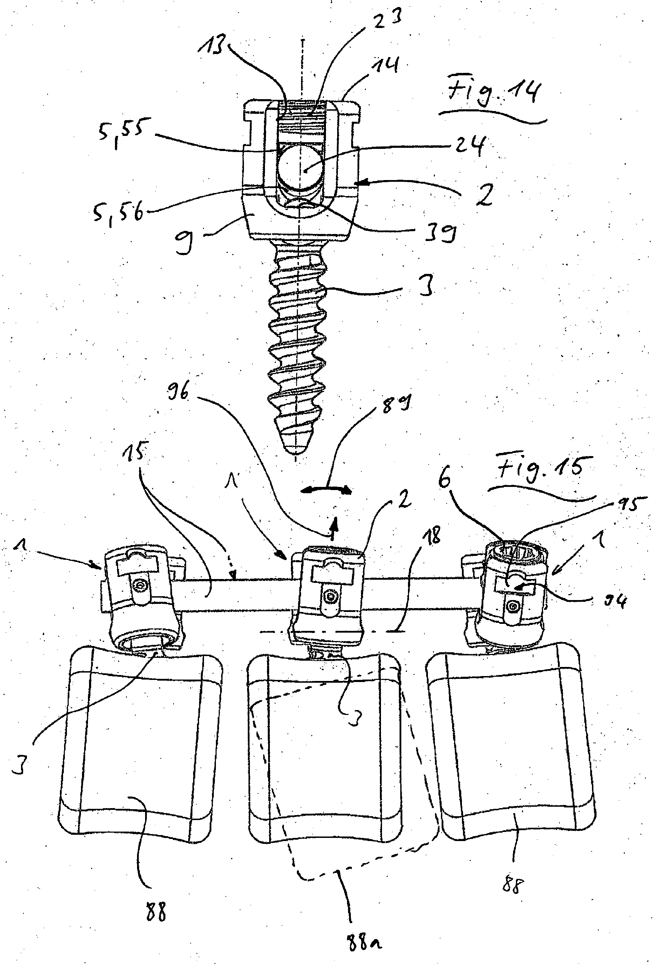

Fig. 15 zeigt eine aus zwei Verbindungsstäben 15 und insgesamt sechs Pedikelschrauben 1 gebildete Vorrichtung zur Stabilisierung der Wirbelsäule. Die Wirbelsäule ist in Fig. 15 durch drei schematisierte Wirbelkörper 88 angedeutet. Bei einer Vorrichtung dieser Art können ausschließlich erfindungsgemäß ausgestaltete Pedikelschrauben 1 verwendet werden. Denkbar ist aber auch die Verwendung von wenigstens einer erfindungsgemäßen Pedikelschraube 1 zusammen mit uniaxialen, polyaxialen oder sonstigen Pedikelschrauben. Da der Schraubenkopf 2 einer Pedikelschraube 1 nur um eine einzige Schwenkachse 18 verschwenkbar ist, kann der Schraubenkopf 2 etwa in Richtung des Doppelpfeiles 89 und damit der Schraubenschaft 3 in der gleichen Richtung verschwenkt und somit ein sich in einer Fehlstellung befindlicher Wirbelkörper 88a reponiert werden.

Dadurch dass das obere Klemmelement 55 so gestaltet ist, dass es drehfixiert im Schraubenkopf 2 gehalten ist, lässt es sich auch mit einem beispielsweise ein rohrförmiges Element 98 aufweisenden Werkzeug 90 einem Schraubenkopf 2 in der richtigen Drehstellung zuführen, d.h. so, dass die Fortsätze 67 in die zwischen den Wandabschnitten 10 des Schraubenkopfs 2 vorhandenen Zwischenräume eingeführt werden. Zu diesem Zweck weist das rohrförmige Element 98 an seiner Innenseite zwei axial verlaufende Nuten 93 auf, in welche die Fortsätze 67 des Klemmelementes 55 formschlüssig hinein passen.

An den Wandabschnitten 10 des Schraubenkopfes 2 ist jeweils eine Ausnehmung 94 vorhanden. Die Ausnehmungen 94 stehen sich diametral gegenüber und weisen einen planeben verlaufenden Grund 95 auf. An den Ausnehmungen 94 kann ein gabelartiges Werkzeug drehfixiert angesetzt und damit eine Fixierschraube 1 bzw. ein damit verbundener Wirbelkörper 88 etwa in Richtung des Pfeils 96 in Fig. 15 reponiert werden.

## Patentansprüche

1. Pedikelschraube (1) mit einem Schraubenschaft (3) und einem damit verbundenen Schraubenkopf (2), der eine sich in einer Richtung, der X-Richtung, erstreckende Mittellängsachse (8) aufweist, mit folgender weiterer Ausgestaltung:
- der Schraubenkopf (2) weist an seinem dem Schraubenschaft zugewandten unteren Ende eine Kopfbasis (9) auf, an der zwei sich vom Schraubenschaft (3) in X-Richtung wegerstreckende Wandabschnitte (10) angeformt sind, welche eine sich in einer rechtwinklig zur X-Richtung erstreckenden Y-Richtung verlaufende, sich in die Kopfoberseite (14) öffnende Ausnehmung (13) zur Aufnahme eines Verbindungsstabs (15) mit kreisrunder Querschnittform seitlich begrenzen,
- der Schraubenschaft ragt über eine mit der Ausnehmung (13) verbundene Bodenöffnung (16) in der Kopfbasis (9) mit einem Gelenkkopf (39) in diese hinein und ist darin derart gelagert, dass der Schraubenkopf (2) gegenüber dem Schraubenschaft (3) um eine einzige, parallel zur Y-Richtung verlaufende Kopfschwenkachse (18) schwenkbar ist,
- in der Ausnehmung (13) ist eine zur Fixierung des Verbindungsstabs (15) im Schraubenkopf (2) dienende Fixierschraube (6) vorhanden, die mit einem Außengewinde (19) in ein an der Innenseite der Wandabschnitte (10) vorhandenes Innengewinde (23) eingreift,
- zwischen der Fixierschraube (6) und der Kopfbasis (9) ist in der Ausnehmung eine Klemmeinrichtung (5) um eine Schwenkachse (25) schwenkbar im Schraubenkopf (2) gehalten, die sich in einer rechtwinklig zur Y- und X-Richtung verlaufenden Z-Richtung erstreckt und die mit Abstand in X-Richtung oberhalb der Kopfschwenkachse (18) angeordnet ist,
- die Klemmeinrichtung (5) weist einen im Montagezustand von dem Verbindungsstab (15) durchsetzten Hohlraum (24) sowie eine der Kopfbasis (9) zugewandte untere Kugelfläche (27a) auf, deren Kugelmittelpunkt auf der Schwenkachse (25) der Klemmeinrichtung liegt,
- die Klemmeinrichtung (5) ist im Montagezustand von der Fixierschraube (6) mit einer zur Kopfbasis (9) gerichteten Kraft beaufschlagt,
**dadurch gekennzeichnet,**
**dass** in der Kopfbasis (9) ein Zwischenelement (4) vorhanden ist, das auf seiner der Klemmeinrichtung (5) zugewandten Seite eine mit der unteren Kugelfläche (27a) der Klemmeinrichtung (5) zusammenwirkende Gegen-Kugelfläche (27b) und auf seiner dem Schraubenschaft (3) zugewandten Seite eine Zylinderfläche (31a) aufweist, die mit einer am Gelenkkopf (39) vorhandenen Gegen-Zylinderfläche (31b) zusammenwirkt, wobei die Zylinderflächen eine gemeinsame, mit der Schwenkachse (18) des Schraubenkopfes (2) zusammenfallende Krümmungsachse (32) aufweisen, und wobei der Gelenkkopf (39) und die Kopfbasis (9) durch einen Bajonettverschluss miteinander verbunden sind.

2. Pedikelschraube nach Anspruch 1,
**dadurch gekennzeichnet,**
**dass** die Bodenöffnung (16) so geformt ist, dass der Gelenkkopf (39) in einer ersten Drehstellung in die Kopfbasis (9) einführbar ist, in einer um 90° verdrehten zweiten Drehstellung dagegen nicht, wobei die Kugelflächen (48a) des sich innerhalb der Kopfbasis (9) befindlichen Gelenkkopfes (39) in der zweiten Drehstellung an den Gegen-Kugelflächen (48b) der Kopfbasis (9) anliegen.

3. Pedikelschraube nach einem der vorhergehenden Ansprüche,
**dadurch gekennzeichnet,**
**dass** der Gelenkkopf (39) zwei diametral gegenüberliegende Kugelflächen (48a) aufweist, die mit komplementären Gegenkugelflächen (48b) an der Wand der Bodenöffnung (16) im Sinne eines Kugelgelenks zusammenwirken.

4. Pedikelschraube nach Anspruch 3,
**dadurch gekennzeichnet,**
**dass** der Gelenkkopf (39) zwei parallel zueinander und in einer von der X- und Z-Richtung aufgespannten Ebene verlaufende Planflächen (40a) aufweist, welche an entsprechend geformten, sich diametral gegenüberliegenden Gegen-Planflächen (40b) an der Wand der Bodenöffnung (16) anliegen.

5. Pedikelschraube nach Anspruch 4,
**dadurch gekennzeichnet,**
**dass** das Zwischenelement (4) zwei nach unten abstehende diametral beabstandete Wandabschnitte (34) aufweist, deren einander zugewandte Innenseiten (35) die Gegen-Planflächen (40b) bilden, wobei die Wandabschnitte (34) in einen zwischen den
Planflächen (40a) und der Wand der Bodenöffnung (16) vorhandenen radial erweiterten Bereich (45) formschlüssig hineinragen.

6. Pedikelschraube nach einem der vorhergehenden Ansprüche,
**dadurch gekennzeichnet,**
**dass** die Schwenkachse (25) der Klemmeinrichtung (5) deren Hohlraum (24) im Montagezustand mittig schneidet.

7. Pedikelschraube nach einem der vorhergehenden Ansprüche,
**dadurch gekennzeichnet,**
**dass** die Klemmeinrichtung (5) ein oberes und ein unteres Klemmelement (55, 56) aufweist, die im Montagezustand konzentrisch zur Schwenkachse (25) der Klemmeinrichtung (5) verlaufende, den Hohlraum (24) zumindest teilweise umgrenzende Klemmflächen (59) aufweisen.

8. Pedikelschraube nach Anspruch 7,
**dadurch gekennzeichnet,**
**dass** das obere Klemmelement (55) ist an der Fixierschraube (6) um deren Mittellängsachse (60) drehbar und axialfest gehalten.

9. Pedikelschraube nach Anspruch 8,
**dadurch gekennzeichnet,**
**dass** das obere Klemmelement (55) eine vom Schraubenschaft (3) weg weisende obere Kugelfläche (63a) und die Fixierschraube (6) eine Gegen-Kugelfläche (63b) aufweist, wobei die genannten Flächen im Montagezustand aneinander anliegen und einen gemeinsamen, auf der Schwenkachse (25) der Klemmeinrichtung (5) liegenden Kugelmittelpunkt aufweisen.

10. Pedikelschraube nach einem der Ansprüche 7 bis 9,
**dadurch gekennzeichnet,**
**dass** das unteren Klemmelement (56) etwa halbringförmig ausgestaltet ist, wobei an seiner im Montagezustand dem Schraubenschaft (3) zugewandten Außenseite die untere Kugelfläche (27a) vorhanden ist.

11. Pedikelschraube nach Anspruch 10,
**dadurch gekennzeichnet,**
**dass** die Freienden des unteren Klemmelements (56) an ihrer Außenseite eine Kugelfläche (77a) aufweisen, die mit GegenKugelflächen (77b) an den Innenseiten der Wandabschnitte (10) des Schraubenkopfes (9) im Sinne einer Gleitpaarung zusammenwirken.

12. Pedikelschraube nach einem der Ansprüche 7 bis 11,
**dadurch gekennzeichnet,**
**dass** die Klemmelemente (55, 56) im Schraubenkopf (2) bezüglich dessen Mittellängsachse (8) drehfixiert gehalten sind.

13. Pedikelschraube nach Anspruch 12,
**dadurch gekennzeichnet,**
**dass** das obere Klemmelement (55) zwei diametral gegenüberliegende Fortsätze (67) aufweist, die mit einem in Umfangsrichtung des Schraubenkopfes (2) wirksamen Formschluss in den zwischen den Wandabschnitten (10) vorhandenen Zwischenraum (68) hineinragen.

14. Pedikelschraube nach Anspruch 12 oder 13,
**dadurch gekennzeichnet,**
**dass** das untere Klemmelement (56) an zwei aus der Innenseite der Wandabschnitte (10) vorstehenden Lagerzapfen (7) schwenkbar gehalten ist.

15. Pedikelschraube nach Anspruch 14,
**dadurch gekennzeichnet,**
**dass** die Lagerzapfen jeweils in ein außenseitig in den Freienden des Klemmelements (56) vorhandenes, sich in Umfangsrichtung erstreckendes Langloch (73) eingreifen.

16. Pedikelschraube nach einem der vorhergehenden Ansprüche,
**dadurch gekennzeichnet,**
**dass** das Innengewinde (23) des Schraubenkopfes (2) einen Gewindezahn (84) mit einer nach unten weisende Flanken (85) und das Außengewinde (19) der Pixierschraube (6) einen Gewindezahn (86) mit einer nach oben weisenden Flanke (87) aufweist, wobei die erstgenannte Flanke (85) mit der Mittellängsachse (8) des schraubenkopfes (2) und die letztgenannte Flanke (87) mit der Mittellängsachse (60) der Fixierschraube (6) einen sich nach oben öffnenden spitzen Winkel (α) einschließen.

17. Vorrichtung zur Stabilisierung der Wirbelsäule, umfassend mehrere Pedikelschrauben und wenigstens einen mit Hilfe der Pedikelschrauben an der Wirbelsäule fixierbaren Verbindungsstab (15), wobei wenigstens eine Pedikelschraube (1) entsprechend einem der vorhergehenden Ansprüche ausgestaltet ist.

## Claims

1. Pedicle screw (1) having a screw shaft (3) and a screw head (2) connected thereto, said screw head (2) having a central longitudinal axis (8) extending in one direction, the X direction, having the following further design:
- the screw head (2) has a head base (9) on its lower end facing the screw shaft, on which head base two wall sections (10) extending away from the screw shaft (3) in the X direction are moulded, said wall sections laterally delimiting a recess (13) which runs in a Y direction extending perpendicularly to the X direction and opens in the head upper side (14) for receiving a connecting rod (15) having a circular cross-sectional shape,
- the screw shaft protrudes into the head base via a bottom opening (16) in the head base (9) connected to the recess (13) with a joint head (39) and is stored therein in such a way that the screw head (2) is able to be pivoted with respect to the screw shaft (3) about a single head pivot axis (18) running parallel to the Y direction,
- a fixing screw (6) serving to fix the connecting rod (15) in the screw head (2) is present in the recess (13), said fixing screw engaging with an inner thread (23) present on the inner side of the wall sections (10) with an outer thread (19),
- in the recess, a clamping device (5) is held in the screw head (2) between the fixing screw (6) and the head base (9) to be pivotable about a pivot axis (25), said clamping device extending in a Z direction which runs perpendicularly to the Y and X directions and being arranged above the head pivot axis (18) with spacing in the X direction,
- the clamping device (5) has a hollow space (24), which is penetrated by the connecting rod (15) in the assembled state, as well as a lower spherical surface (27a) facing the head base (9), the sphere centre of which rests against the pivot axis (25) of the clamping device,
- the clamping device (5) is applied with a force directed towards the head base (9) by the fixing screw (6) in the assembled state,
**characterised in that**
an intermediate element (4) is present in the head base (9), said intermediate element having a counter spherical surface (27b) on its side facing the clamping device (5) which interacts with the lower spherical surface (27a) of the clamping device (5) and having a cylindrical surface (31a) on its side facing the screw shaft (3), said cylindrical surface interacting with a counter cylindrical surface (31b) which is present on the joint head (39), wherein the cylindrical surfaces have a common axis of curvature (32) which coincides with the pivot axis (18) of the screw head (2) and wherein the joint head (39) and the head base (9) are connected to each other by a bayonet catch.

2. Pedicle screw according to claim 1,
**characterised in that**
the bottom opening (16) is formed in such a way that the joint head (39) is able to be introduced into the head base (9) in a first rotational position, however not in a second rotational position rotated by 90°, wherein the spherical surfaces (48a) of the joint head (39) located within the head base (9) rest against the counter spherical surfaces (48b) of the head base (9) in the second rotational position.

3. Pedicle screw according to one of the preceding claims,
**characterised in that**
the joint head (39) has two diametrically opposite spherical surfaces (48a) which interact with complementary counter spherical surfaces (48b) on the wall of the bottom opening (16) in the manner of a ball joint.

4. Pedicle screw according to claim 3,
**characterised in that**
the joint head (39) has two plane surfaces (40a) which run in parallel with each other and in a plane stretched from the X and Z directions and which rest against correspondingly formed, diametrically opposite counter plane surfaces (40b) on the wall of the bottom opening (16).

5. Pedicle screw according to claim 4,
**characterised in that**
the intermediate element (4) has two diametrically spaced wall sections (34) which protrude downwards, the inner sides (35) of which facing each other forming the counter plane surfaces (40b), wherein the wall sections (34) protrude positively in a radially expanded region (45) which is present between the plane surfaces (40a) and the wall of the bottom opening (16).

6. Pedicle screw according to one of the preceding claims,
**characterised in that**
the pivot axis (25) of the clamping device (5) intersects its hollow space (24) centrally in the assembled state.

7. Pedicle screw according to one of the preceding claims,
**characterised in that**
the clamping device (5) has an upper and a lower clamping element (55, 56) which have clamping surfaces (59) running concentrically to the pivot axis (25) of the clamping device (5) in the assembled state and at least partially surrounding the hollow space (24).

8. Pedicle screw according to claim 7,
**characterised in that**
the upper clamping element (55) is held on the fixing screw (6) to be able to be rotated about its central longitudinal axis (60) and in an axially fixed manner.

9. Pedicle screw according to claim 8,
**characterised in that**
the upper clamping element (55) has an upper spherical surface (63a) facing away from the screw shaft (3) and the fixing screw (6) has a counter spherical surface (63b), wherein said surfaces rest against each other in the assembled state and have a common sphere centre resting against the pivot axis (25) of the clamping device (5).

10. Pedicle screw according to one of claims 7 to 9,
**characterised in that**
the lower clamping element (56) is designed to be approximately semi-annular, wherein the lower spherical surface (27a) is present on its outer side facing the screw shaft (3) in the assembled state.

11. Pedicle screw according to claim 10,
**characterised in that**
the free ends of the lower clamping element (56) have a spherical surface (77a) on their outer side, said spherical surfaces interacting with counter spherical surfaces (77b) on the inner sides of the wall sections (10) of the screw head (9) in the manner of a pair of sliding elements.

12. Pedicle screw according to one of claims 7 to 11,
**characterised in that**
the clamping elements (55, 56) are held in the screw head (2) in a rotationally fixed manner with respect to its central longitudinal axis (8).

13. Pedicle screw according to claim 12,
**characterised in that**
the upper clamping element (55) has two diametrically opposite extensions (67) which protrude into the intermediate space (68) which is present between the wall sections (10) with a positive connection acting in the peripheral direction of the screw head (2).

14. Pedicle screw according to claim 12 or 13,
**characterised in that**
the lower clamping element (56) is held pivotably on two bearing journals (7) protruding from the inner side of the wall sections (10).

15. Pedicle screw according to claim 14,
**characterised in that**
the bearing journals each engage with an external slot (73) which is present in the free ends of the clamping element (56) and extends in the peripheral direction.

16. Pedicle screw according to one of the preceding claims,
**characterised in that**
the internal thread (23) of the screw head (2) has a thread tooth (84) having an edge (85) facing downwards and the outer thread (19) of the fixing screw (6) has a thread tooth (86) having an edge (87) facing upwards, wherein the first-mentioned edge (85) forms an upwardly opening acute angle (α) with the central longitudinal axis (8) of the screw head (2) and the last-mentioned edge (87) forms an upwardly opening acute angle (α) with the central longitudinal axis (60) of the fixing screw (6).

17. Device for stabilising the spinal column, comprising several pedicle screws and at least one connecting rod (15) which is able to be fixed to the spinal column with the aid of the pedicle screws, wherein at least one pedicle screw (1) is designed in accordance with one of the preceding claims.

## Revendications

1. Vis (1) pédiculaire ayant une tige (3) et une tête (2) qui y est reliée et qui a un axe (8) longitudinal médian s'étendant dans une direction, la direction X, ayant la conformation supplémentaire suivante :
- la tête (2) de la vis a, à son extrémité inférieure, tournée vers la tige de la vis, une base (9) de tête sur laquelle sont formées deux parties (10) de paroi s'éloignant de la tige (3) de la vis dans la direction X et délimitant latéralement pour la réception d'une barre (15) de liaison de forme de section transversale circulaire, un évidement (13) s'étendant dans une direction Y à angle droit avec la direction X et s'ouvrant dans le côté (14) supérieur de la tête,
- la tige de la vis dépasse une ouverture (16) de fond, communiquant avec l'évidement (13) dans la base de la tête, par une tête (39) d'articulation y pénétrant et y est montée, de manière à ce que la tête (2) de la vis puisse pivoter par rapport à la tige (3) de la vis autour d'un axe (18) de pivotement de tête unique, s'étendant parallèlement à la direction Y,
- il y a dans l'évidement (13) une vis (6) d'immobilisation servant à immobiliser la barre (15) de liaison dans la tête (2) de la vis et pénétrant par un filetage (19) dans un taraudage (23) présent sur le côté intérieur des parties (10) de paroi,
- entre la vis (6) d'immobilisation et la base (9) de la tête est maintenu dans la tête (2) de la vis, pivotant autour d'un axe (25) de pivotement dans l'évidement, un dispositif (5) de serrage, qui s'étend dans une direction Z à angle droit avec la direction X et la direction Y et qui est disposé à distance dans la direction X au-dessus de l'axe (18) de pivotement de la tête,
- le dispositif (5) de serrage a une cavité (24) traversée, à l'état de montage, par la barre (15) de liaison, ainsi qu'une surface (27a) sphérique inférieure tournée vers la base (9) de la tête et dont le centre de sphère se trouve sur l'axe (25) de pivotement du dispositif de serrage,
- le dispositif (5) de serrage est, à l'état monté, soumis par la vis (6) d'immobilisation, à une force dirigée vers la base (9) de la tête,
**caractérisée,**
**en ce qu'**il y a dans la base (9) de la tête un élément (4) intermédiaire qui a, du côté tourné vers le dispositif (5) de serrage, une surface (27b) sphérique antagoniste coopérant avec la surface (25a) sphérique inférieure du dispositif (5) de serrage et, du côté tourné vers la tige (3) de la vis, une surface (31a) cylindrique qui coopère avec une surface (31b) cylindrique antagoniste présente sur la tête (39) d'articulation, les surfaces cylindriques ayant un axe (32) de courbure commun coïncidant avec l'axe (18) de pivotement de la tête (2) de la vis et la tête (39) d'articulation et la base (9) de la tête étant reliées entre elles par une fermeture à baïonnette.

2. Vis pédiculaire suivant la revendication 1,
**caractérisée,**
**en ce que** l'ouverture (16) du fond est conformée de manière à ce que la tête (39) d'articulation puisse être introduite en une première position en rotation dans la base (9) de la tête, mais non en revanche dans une deuxième position en rotation tournée de 90°, les surfaces (48a) sphériques de la tête (39) d'articulation, se trouvant à l'intérieur de la base (9) de la tête, s'appliquant, dans la deuxième position en rotation, aux surfaces (48b) sphériques antagonistes de la base (9) de la tête.

3. Vis pédiculaire suivant l'une des revendications précédentes,
**caractérisée en ce que**
la tête (39) d'articulation a deux surfaces (48a) sphériques opposées diamétralement qui coopèrent au sens d'une rotule avec des surfaces (48b) antagonistes complémentaires sur la paroi de l'ouverture (16) du fond.

4. Vis pédiculaire suivant la revendication 3,
**caractérisée en ce que**,
la tête (39) d'articulation a deux surfaces (40a) planes s'étendant parallèlement entre elles et dans un plan passant par la direction X et par la direction Z, surfaces qui s'appliquent à des surfaces (40b) planes antagonistes opposées diamétralement, conformées de manière correspondante sur la paroi de l'ouverture (16) du fond.

5. Vis pédiculaire suivant la revendication 4,
**caractérisée en ce que**
l'élément (4) intermédiaire a deux parties (34) de paroi qui sont à distance diamétralement en saillie vers le bas et dont les côtés (35) intérieurs tournés l'un vers l'autre, forment les surfaces (40b) planes antagonistes, les parties (14) de paroi pénétrant à complémentarité de forme dans une région (45) élargie radialement, présente entre les surfaces (40a) planes et la paroi de l'ouverture (16) du fond.

6. Vis pédiculaire suivant l'une des revendications précédentes,
**caractérisée en ce que**
l'axe (25) de pivotement du dispositif (5) de serrage coupe au milieu, à l'état monté, la cavité (24) du dispositif (5) de serrage.

7. Vis pédiculaire suivant l'une des revendications précédentes,
**caractérisée**
**en ce que** le dispositif (5) de serrage a un élément (55) supérieur de serrage et un élément (56) inférieur de serrage qui ont, à l'état monté, des surfaces (59) de serrage s'étendant concentriquement à l'axe (25) de pivotement du dispositif (5) de serrage et entourant, au moins en partie, la cavité (24).

8. Vis pédiculaire suivant la revendication 7,
**caractérisée en ce que**
l'élément (55) supérieur de serrage est sur la vis (6) de fixation maintenu tournant autour de l'axe (60) longitudinal médian de celle-ci et fixe axialement.

9. Vis pédiculaire suivant la revendication 8,
**caractérisée en ce que**
l'élément (55) supérieur de serrage a une surface (63) sphérique supérieure éloignée de la tige (3) de la vis et la vis (6) de fixation a une surface (63b) sphérique antagoniste, lesdites surfaces s'appliquant l'une à l'autre à l'état monté, et ayant un centre de sphère commun se trouvant sur l'axe (25) de pivotement du dispositif (5) de serrage.

10. Vis pédiculaire suivant l'une des revendications 7 à 9,
**caractérisé en ce que**
l'élément élément (56) inférieur de serrage est en forme de demi-bague, la surface (27a) sphérique inférieure étant présente sur son côté extérieur tourné, à l'état monté, vers la tige (3) de la vis.

11. Vis pédiculaire suivant la revendication 10,
**caractérisée en ce que**
les extrémités libres de l'élément (56) inférieur de serrage ont, sur leur côté extérieur, une surface (77a) sphérique qui coopère au sens d'un appariement glissant avec des surfaces (77b) sphériques antagonistes sur les côtés intérieurs des parties (10) de paroi de la tête (9) de la vis.

12. Vis pédiculaire suivant l'une des revendications 7 à 11,
**caractérisée en ce que**
les éléments (55, 56) de serrage sont dans la tête (2) de la vis maintenus immobilisés en rotation par rapport à son axe (8) longitudinal médian.

13. Vis pédiculaire suivant la revendication 12,
**caractérisée en ce que**
l'élément (55) supérieur de serrage a deux prolongements (67) opposés diamétralement, qui pénètrent par une complémentarité de forme efficace dans la direction périphérique de la tête (2) de la vis, dans l'espace (68) intermédiaire présent entre les parties (10) de paroi.

14. Vis pédiculaire suivant la revendication 12 ou 13,
**caractérisée en ce que** ,
l'élément (56) inférieur de serrage est maintenu pivotant sur deux tourillons (7) en saillie du côté intérieur des parties (10) de paroi.

15. Vis pédiculaire suivant la revendication 14,
**caractérisée en ce que**
les tourillons pénètrent respectivement dans une boutonnière (73) présente du côté extérieur dans les extrémités libres de l'élément (56) de serrage et s'étendant dans la direction périphérique.

16. Vis pédiculaire suivant l'une des revendications précédentes,
**caractérisée en ce que**
le taraudage (23) de la tête (2) de la vis a une denture (84) filetée ayant un flanc (85) tourné vers le bas, et le filetage (19) de la vis (6) d'immobilisation a une denture (86) ayant un flanc (87) tourné vers le haut, le flanc (85) mentionné en premier faisant, avec l'axe (8) longitudinal médian de la tête (2) de la vis et le flanc (87) mentionné en dernier avec l'axe (60) longitudinal médian de la vis (6) d'immobilisation, un angle (α) aigu s'ouvrant vers le haut.

17. Dispositif de stabilisation de la colonne vertébrale comprenant plusieurs vis pédiculaires et au moins une barre (15) de liaison pouvant être immobilisée sur la colonne vertébrale à l'aide des vis pédiculaires, au moins l'une des vis (1) pédiculaires étant conformée conformément à l'une des revendications précédentes.
